# EUROPEAN PATENT SPECIFICATION

(11) **EP 0 474 303 B1**
(45) Date of publication and mention of the grant of the patent: **17.01.1996**
(21) Application number: 91202228.2
(22) Date of filing: 02.09.1991
(51) Int. Cl.: C07C 35/21

(54) **Process for the preparation of cyclopentane derivatives and intermediates therefor**
Herstellung von Cylopentan-Derivaten und Zwischenprodukte dafür
Procédé de préparation de dérivés cyclopentane et produits intermédiaires employés

(30) Priority: 03.09.1990 GB 9019191; 03.09.1990 GB 9019190
(43) Date of publication of application: 11.03.1992
(73) Proprietor: SHELL INTERNATIONALE RESEARCH MAATSCHAPPIJ B.V., NL-2596 HR Den Haag (NL)
(72) Inventor: Briner, Paul Howard, Molash, Canterbury, Kent CT4 8EY (GB)
(74) Representative: Allam, Peter Clerk

(56) References cited:
- EP-A- 0 267 778
- EP-A- 0 359 305
- GB-A- 2 180 236
- JUSTUS LIEBIGS ANNALEN DER CHEMIE, vol. 414, 1918, pages 296-366, Weinheim, DE; O. WALLACH, "Zur Erkenntnis der Terpene und der ätherischen Öle"
- CHEMICAL ABSTRACTS, vol. 75, no. 15, 11 October 1971, page 271, left column, abstract no. 98009f, Columbus, Ohio, US; J.M. CONIA et al, "Cyclobutane-1,2-dione. Ring contraction by water"

## Description

This invention relates to a process for the preparation of cyclopentanediol derivatives via cyclopentanol derivative intermediates, the cyclopentanol derivatives per se and a process for their preparation. The cyclopentanediol and cyclopentanol derivatives are useful as intermediates in the preparation of certain fungicidally active cyclopentane derivatives.

According to the present invention there is provided a process for the preparation of a compound of the general formula 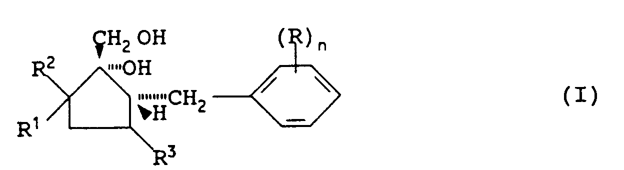
in which n represents an integer from 0 to 5; each R represents a halogen atom, nitro, cyano, hydroxyl, alkyl, haloalkyl, alkoxy, haloalkoxy, amino, alkylamino, dialkylamino, alkoxycarbonyl, carboxyl, alkanoyl, alkylthio, alkylsulphinyl, alkylsulphonyl, carbamoyl, alkylamido, cycloalkyl or phenyl group; and R¹, R² and R³ independently represent a hydrogen atom or an alkyl group; which comprises reacting a compound of the general formula 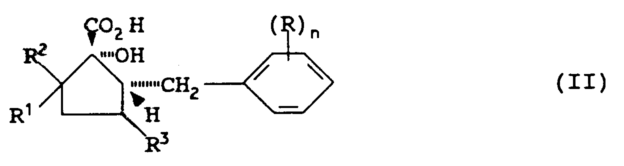
in which n, R, R¹, R² and R³ are as defined above, with a reducing agent.

It is preferred that the reducing agent is a complex metal hydride, such as lithium aluminium hydride, "REDAL" (Trade Mark: sodium bis(2-methoxyethoxy)aluminium hydride in toluene) or sodium borohydride in the presence of aluminium chloride.

The process is conveniently carried out in the presence of a solvent. Suitable solvents include ethers, such as diethyl ether and diglyme, and hydrocarbons, such as toluene.

Preferably, the reaction is carried out at a temperature from 0°C to the reflux temperature of the solvent depending on the nature of the reducing agent selected.

It is advisable to destroy any excess reducing agent remaining at the end of the reduction process to prevent further reaction. If a complex metal hydride, such as lithium aluminium hydride, is used as reducing agent, any excess may be destroyed by the addition of water and sodium hydroxide or ammonium chloride to the reaction mixture.

According to the present invention there is provided a compound of the general formula 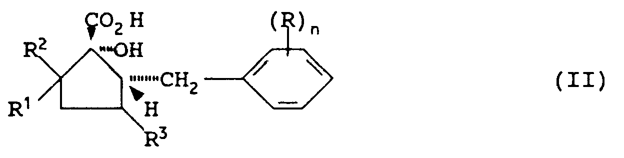
in which n, R, R¹, R² and R³ are as previously defined.

The present invention further provides a process for the preparation of a compound of formula II as defined above which comprises heating a compound of the general formula 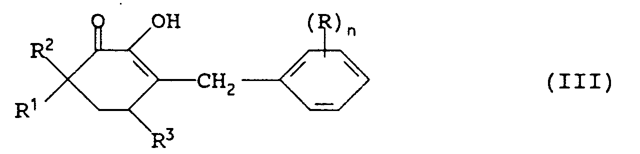
or the general formula 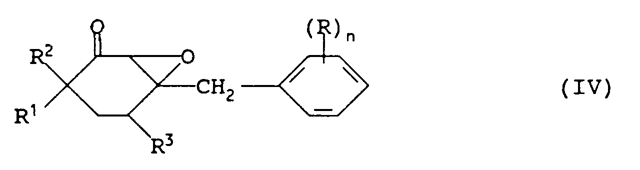
in which n, R, R¹, R² and R³ are as defined above, with an alkali metal hydroxide, especially sodium or potassium hydroxide, in the presence of a polar solvent.

Preferably, the polar solvent is a high boiling oligoether, such as 2-(2-methoxyethoxy)ethanol, or an alcohol, preferably a C₁₋₆ and especially a C₄₋₆ tertiary alcohol. The reaction is conveniently carried out at a temperature from 130°C to the reflux temperature of the solvent, preferably from 135° to 155°C.

Compounds of formula III may be conveniently prepared by reacting a compound of formula IV in which n, R, R¹, R² and R³ are as defined above, with a compound of the general formula

MOR⁵ (V)

in which R⁵ represents a hydrogen atom or an alkyl, preferably a C₁₋₆ tertiary alkyl and especially a C₄₋₆ tertiary alkyl, or cycloalkyl, preferably a C₃₋₆ cycloalkyl, group and M represents an alkali metal, preferably a sodium or potassium, atom in the presence of a polar solvent. The compounds of formula III and a process for their preparation form the subject of copending patent application T 690.

Compounds of formula IV may be conveniently prepared by reacting a compound of formula 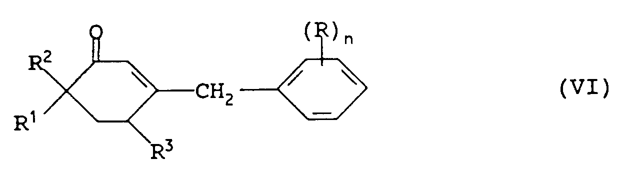
in which n, R, R¹, R² and R³ are as defined above, with hydrogen peroxide in the presence of a base, preferably an inorganic base such as sodium hydroxide, potassium hydroxide or a quaternary ammonium hydroxide. The compounds of formula IV and a process for their preparation form the subject of copending patent application T 690.

Compounds of formula VI may be conveniently prepared by reacting a compound of formula 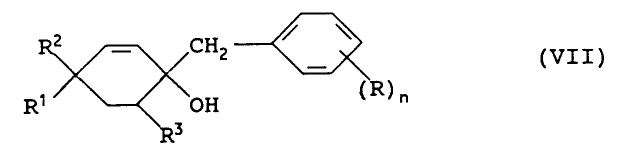
in which n, R, R¹, R² and R³ are as defined above, with an oxidising agent, preferably a chromium (VI) salt such as an alkali metal dichromate, particularly sodium dichromate or potassium dichromate. The compounds of formula VI and a process for their preparation form the subject of copending patent application T 690.

Compounds of formula VII may be conveniently prepared by reacting a compound of formula 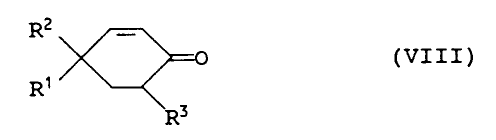
in which R¹, R² and R³ are as defined above, with a compound of the general formula 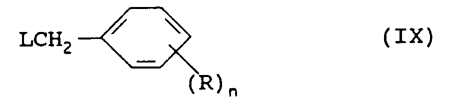
in which R and n are as defined above and L represents an organometallic group, such as lithium or the group -MgHal where Hal represents a chlorine or bromine atom. The compounds of formula VII and a process for their preparation form the subject of copending patent application T 689.

Compounds of formula V, VIII and IX are known compounds or can be prepared by processes analogous to known processes.

When any of the foregoing substituents represents or contains an alkyl substituent group, this may be linear or branched and may contain up to 12, preferably up to 6, and especially up to 4 carbon atoms. A cycloalkyl substituent group may contain 3 to 8, preferably 3 to 6, carbon atoms.

It is preferred that R¹, R² and R³ independently represent a hydrogen atom or a C₁₋₄ alkyl, particularly a methyl group.

Preferably, R represents a halogen, especially a chlorine atom.

A particularly preferred sub-group of compounds of formulae I and II is that in which n is 1, R represents a chlorine atom, preferably substituted at the 4- position of the phenyl ring, R¹ and R² both represent a hydrogen atom or both represent a methyl group and R³ represents a hydrogen atom or methyl group.

The compounds of formula I are useful as intermediates in the preparation of fungicidally active cyclopentane derivatives of the general formula 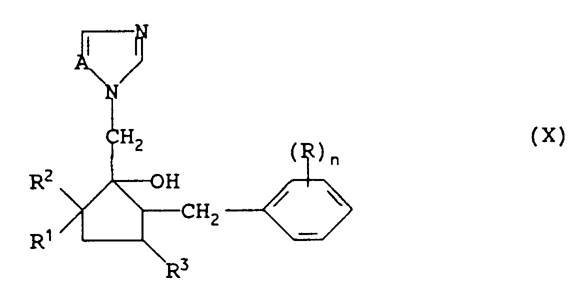
in which n, R, R¹, R² and R³ are as defined above and A represents a nitrogen atom or a CH group. Certain compounds of formula X are the subject of co-pending patent applications GB-Al-2180236 and EP-A2-0267778. The compounds disclosed in EP-A2-0267778 and GB-Al-2180236 exist in two stereoisomeric forms which have the following structures:- 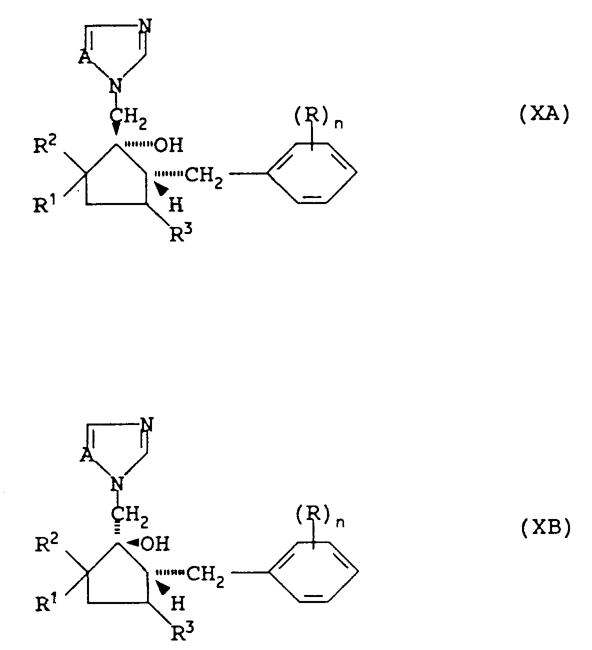
The letters A and B will be used hereinafter to denote compounds having the same stereochemical configuration as isomers A and B above.

Isomers A and B can be separated by, for instance, chromatography and exhibit different fungicidal activity. Generally, isomers of formula XA exhibit greater fungicidal activity than isomers of formula XB. The process used to synthesise compounds of formula XA from compounds of formula I is set out in the following reaction scheme:-

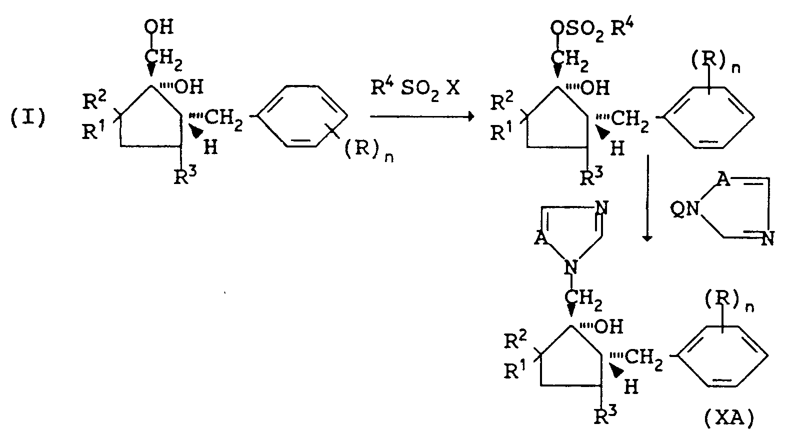

In the above reaction scheme, n, R, R¹, R², R³, M and A are as previously defined, R⁴ represents an optionally substituted alkyl or aryl group, preferably a C₁₋₄alkyl or a phenyl group each optionally substituted by one or more substituents selected from halogen atoms, nitro, cyano, hydroxyl, C₁₋₄alkyl, C₁₋₄haloalkyl, C₁₋₄alkoxy, C₁₋₄haloalkoxy, amino, C₁₋₄alkylamino, di-C₁₋₄alkylamino, C₁₋₄alkoxycarbonyl, carboxyl, C₁₋₄alkanoyl, C₁₋₄alkylthio, C₁₋₄alkylsulphinyl, C₁₋₄alkylsulphonyl, carbamoyl, C₁₋₄alkylamido, C₃₋₈cycloalkyl and phenyl groups, X represents a halogen, preferably a chlorine or bromine, atom and Q represents a hydrogen or alkali metal, preferably sodium or potassium, atom. The compounds of formula I per se and the intermediate compounds and process steps in the above reaction scheme are the subject of copending European patent application no 89202159.3 and copending British patent application no. 8820607.3.

The invention is further illustrated by the following Examples.

### Example 1

### Preparation of 1-(4-chlorobenzyl)-2-carboxyl-2-hydroxy-3,3-dimethylcyclopentane (Formula II: n=1, R=4-Cl, R¹=R²=CH₃, R³=H)

(a) Preparation of 1-(4-chlorobenzyl)-4,4-dimethylcyclohex-2-en-1-ol
   A solution of 4-chlorobenzyl chloride (266g, 1.65mol) in diethyl ether (200ml) was added slowly to a stirred mixture of magnesium (42g, 1.73mol) in diethyl ether (700ml) to maintain the mixture at reflux. The mixture was warmed for a further 20 minutes after addition was complete. A solution of 4,4-dimethylcyclohex- 2-en-1-one (226g, 1.82 mol) in diethyl ether (60ml) was then added dropwise over a period of 30 minutes so as to maintain the mixture at reflux and the mixture stirred overnight. The mixture was then quenched with water (250ml) and hydrochloric acid (5M, 500ml), extracted with diethyl ether (3x400ml), backwashed once with sodium bicarbonate solution (5%w/v) and once with water and then dried with anhydrous magnesium sulphate. The solvent was then flashed off to give 369g 1-(4-chlorobenzyl)-4,4-dimethylcyclohex-2-en-1-ol as an oil.
   NMR (in CDCl₃ solvent, tetramethylsilane as reference) Characteristic peaks at:-
   - δ(ppm): 0.90, 0.99 (3H, singlet), 2.78 (2H, singlet), 5.40 (1H, doublet, J=11Hz), 5.50 (1H, doublet, J=11Hz), 7.17 (2H, doublet, J=8Hz), 7.26 (2H, doublet, J=8Hz).
(b) Preparation of 1-(4-chlorobenzyl)-4,4-dimethylcyclohex-1-en-3-one
   A solution of the 1-(4-chlorobenzyl)-4,4-dimethylcyclohex-2-en-1-ol (368g, 1.47mol) obtained in (a) in 40/60 petroleum (40ml) was added in a steady stream to a solution of sodium dichromate (217g, 0.74mol) in dilute sulphuric acid (250g, 2.6mol 98% sulphuric acid in 1.5 litres of water). The reaction mixture was then held at a temperature between 10° and 30°C and stirred for 40 minutes. Water (500ml) and diethyl ether (700ml) were added and the aqueous layer extracted twice with diethyl ether (2 x 700ml). The organic phases were then combined and backwashed with saturated sodium bicarbonate solution (1 x 500ml) and water (1 x 500ml). The solvent was then flashed off to give 349g crude 1-(4-chlorobenzyl)-4,4-dimethylcyclohex-1-en-3-one as a beige coloured granular solid.
   Trituration in petrol gave a pure sample of the desired product, m.pt. 87-90°C.
(c) Preparation of 1-(4-chlorobenzyl)-1,2-epoxy-4,4-dimethylcyclohexan-3-one
   1726g (6.945mol) crude 1-(4-chlorobenzyl)-4,4-dimethylcyclohex-1-en-3-one obtained as described in (b) above and ethanol (8630ml) were charged into a 20 litre reactor and warmed to 40°C to give a clear pale orange solution. The reaction mixture was then cooled to 18°C and 20% (w/v) sodium hydroxide (650ml) was added slowly with cooling (ice/water) to maintain this temperature. Keeping the reaction mixture at a temperature between 11° and 20°C, 30%(w/v) aqueous hydrogen peroxide (794ml, 7mol) was added over a period of 1 hour and the mixture was then stirred overnight. Water (16 litres) was then added with ice cooling and the reaction mixture stirred for 15 minutes. Centrifugation followed by washing with water (4 x 2½ litres) yielded an off-white solid which was then air dried to give 1634g 1-(4-chlorobenzyl)-1,2-epoxy-4,4-dimethylcyclohexan-3-one, m.pt. 69°-70°C.
(d) Preparation of 1-(4-chlorobenzyl)-2-carboxyl-2-hydroxy-3,3-dimethylcyclopentane
   1000g (3.78mol) Crude 1-(4-chlorobenzyl)-1,2-epoxy-4,4-dimethylcyclohexan-3-one obtained in (c) above was dissolved in tert-butanol (4200ml) at 40°-50°C and the temperature raised to 55°C. Solid potassium hydroxide (2 x 125g portions) was added and the reaction mixture was then left for 1½ hours. 2-(2-Methoxyethoxy)ethanol (4200ml) was then gradually added whilst the reaction mixture was heated to 150°C for 6½ hours to remove the tert-butanol by short column distillation (4 litres recovered). The mixture was then cooled overnight and 4.5M hydrochloric acid (3.5 litres) was added with dry ice/acetone cooling to keep the reaction mixture at 20°C. The mixture was then stirred for 30 minutes, centrifuged and washed with water (4 litres). The solid residue was then taken into toluene, azeotropically dried, cooled overnight, centrifuged and washed with 60/80 petroluem (2 litres) to give 655g 1-(4-chlorobenzyl)-2-carboxyl-2-hydroxy-3,3-dimethylcyclopentane as a white crystalline solid, m.pt. 157°-158°C.

### Example 2

### Preparation of 1-(4-chlorobenzyl)-2-hydroxy-2-hydroxymethyl-3,3-dimethylcyclopentane (Formula I: n=1, R=4-Cl, R¹=R²=CH₃, R³=H)

A 35% solution of "REDAL" (Trade Mark: sodium bis(2-methoxyethoxy) aluminium hydride in toluene) (5ml, 1.25 equivalents active hydrogen) was added to a suspension of 1-(4-chlorobenzyl)-2-carboxyl-2-hydroxy-3,3-dimethylcyclopentane (1g) obtained in Example 1 above at 0°-5°C. The reaction mixture was then heated to 60-70°C overnight and worked up by backwashing with 5M hydrochloric acid. The mixture was then reextracted and the phases combined, washed twice with water and the solvent flashed off to give 0.8g 1-(4-chlorobenzyl)-2-hydroxy-2-hydroxymethyl-3,3-dimethylcyclopentane as a solid, m.pt. 103°-104°C.

### Example 3

### Preparation of 1-(4-chlorobenzyl)-2-carboxyl-2-hydroxy-3,3,5-trimethylcyclopentane (Formula II: n=1,R=4-Cl, R¹=R²=R³=CH₃)

(a) Preparation of 1-(4-chlorobenzyl)-4,4,6-trimethylcyclohex-2-en-1-ol
   To a slurry of magnesium turnings (66g, 2.73 g atoms) in diethyl ether (300ml) was added a solution of 4-chlorobenzyl chloride (418g, 2.6 moles) in diethyl ether (1500ml) at such a rate as to maintain gentle reflux. After a further 30 minutes, a solution of 4,4,6-trimethylcyclohex-2-en-1-one (340g, 2.46 moles) in diethyl ether (350ml) was added, again maintaining a gentle reflux. After 1 hour the mixture was added into saturated aqueous ammonium chloride (4 litres) and the phases separated. The ether phase was back-washed with water (1 litre) and used directly in the next reaction. A small portion of 1-(4-chlorobenzyl)-4,4,6-trimethylcyclohex-2-en-1-ol was isolated for characterisation (gas chromatography analysis showed two isomers in approximately equal amounts).
   NMR (in CDCl₃ solvent, tetramethylsilane as reference). Characteristics peak at:-
   - δ (ppm): 0.75, 0.95, 1.00, 1.02, 1.05, 1.07, 1.09 (total 9H), 2.00 (1H,multiplet), 2.57, 2.79 (2H, AB, J=12Hz), 2.69, 2.94 (2H, AB, J=12Hz), 4.94 (1H, doublet, J=10Hz), 5.34 (1H, doublet, J=10Hz), 7.1-7.4 (4H).
(b) Preparation of 1-(4-chlorobenzyl)-4,4,6-trimethylcyclohex-1-en-3-one
   A solution of sodium dichromate (281g, 0.943mol) in dilute sulphuric acid (428g 98% sulphuric acid in 2.5 litres of water) was added to the ethereal solution of 1-(4-chlorobenzyl)-4,4,6-trimethylcyclohex-2-en-1-ol obtained in (a). The reaction mixture was then heated to 50-60°C for 3-4 hours, cooled and quenched with water (2 litres) and diethyl ether (1 litre). The phases were separated and the organic phase was washed with 20% (w/v) sodium hydroxide (2 x 500ml) to give a clear pale brown solution. Stripping off the solvent gave a mixture of crystalline solid and oily liquid which was then triturated in 60/80 petroleum (1 litre) at 0°C and filtered to give 315g 1-(4-chlorobenzyl)-4,4,6-trimethylcyclohex-1-en-3-one as a crystalline white solid, m.pt. 76°-77°C.
(c) Preparation of 1-(chlorobenzyl)-1,2-epoxy-4,4,6-trimethylcyclohexan-3-one
   The 1-(chlorobenzyl)-4,4,6-trimethylcyclohexan-3-one (315g, 1.2mol) obtained in (b) above was added to methanol (1500ml) and the mixture warmed to 40°C to give a clear yellow solution. The solution was then cooled to 10°C and aqueous sodium hydroxide (25g in 112ml water) was added over a period of 10 minutes. Whilst maintaining the temperature of the reaction mixture between 15° and 20°C, 30% (w/v) aqueous hydrogen peroxide (138ml, 1.2mol) was added over a period of 30 minutes and the mixture was then stirred overnight. A further 30ml was then added and the mixture stirred for 2½ hours. The reaction mixture was then concentrated under reduced pressure and diluted with water (2 litres) and diethyl ether (1.5 litres). The aqueous layer was then extracted with diethyl ether (2 x 0.5 litres), dried and the solvent flashed off to give 1-(4-chlorobenzyl)-1,2-epoxy-4,4,6-trimethylcyclohexan-3-one as a crystalline white solid (271g), m.pt. 58°-59°C.
(d) Preparation of 1-(4-chlorobenzyl)-2-carboxyl-2-hydroxy- 3,3,5-trimethylcyclopentane
   Flake potassium hydroxide (30.4g, 3 equivalents) was added to a solution of the 1-(4-chlorobenzyl)-1,2-epoxy-4,4,6-trimethylcyclohexan-3-one (43g, 0.154mol) obtained in (c) above in tert-butanol (200ml) at 40-50°C and the mixture brought to relux for 3 hours. A solvent switch to 2-(2-methoxyethoxy)ethanol (150ml) was effected by distillation of tert-butanol through a short column whilst dosing the 2-(2-methoxyethoxy)ethanol. The temperature was gradually raised to 140°-150°C and held at that temperature for 6 hours. The reaction mixture was then poured into ice-cold 2M hydrochloric acid (400ml) and the solid filtered, washed with water and dissolved in refluxing toluene (150ml) under a Dean-Stark separator. The mixture was then cooled, refiltered, washed with toluene then 40/60 petroluem and dried to give 34g 1-(4-chlorobenzyl)-2-carboxyl-2-hydroxy-3,3-5-trimethylcyclopentane as a white solid, m.pt. 170°-171°C.

### Example 4

### Preparation of 1-(4-chlorobenzyl)-2-hydroxy-2-hydroxymethyl-3,3,5-trimethylcyclopentane (Formula I: n=1, R=4-Cl, R¹=R²=R³=CH₃)

34g 1-(4-chlorobenzyl)-2-carboxyl-2-hydroxy-3,3,5-trimethylcyclopentane obtained in Example 3 above was added to a suspension of lithium aluminium hydride (17g) in diethyl ether (200ml) and the mixture refluxed overnight. The mixture was worked up with water (17ml), 15% (w/v) sodium hydroxide (17ml) and more water (51ml) and the solvent flashed off to give 32g 1-(4-chlorobenzyl)-2-hydroxy-2-hydroxymethyl-3,3,5-trimethylcyclopentane as a white solid, m.pt. 93°-94°C.

## Claims

1. A process for the preparation of a compound of the general formula 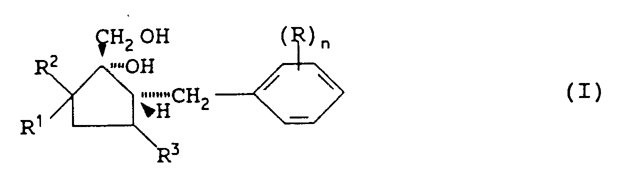 in which n represents an integer from 0 to 5; each R represents a halogen atom, nitro, cyano, hydroxyl, alkyl, haloalkyl, alkoxy, haloalkoxy, amino, alkylamino, dialkylamino, alkoxycarbonyl, carboxyl, alkanoyl, alkylthio, alkylsulphinyl, alkylsulphonyl, carbamoyl, alkylamido, cycloalkyl or phenyl group; and R¹, R² and R³ independently represent a hydrogen atom or an alkyl group; which comprises reacting a compound of the general formula 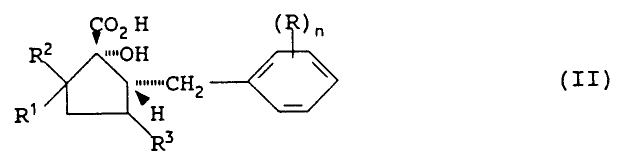 in which n, R, R¹, R² and R³ are as defined above, with a reducing agent.

2. A process according to claim 1 in which the reducing agent is lithium aluminium hydride or sodium bis(2-methoxyethoxy)aluminium hydride.

3. A compound of the general formula II as defined in claim 1.

4. A process for the preparation of a compound of formula II as defined in claim 1 which comprises heating a compound of the general formula 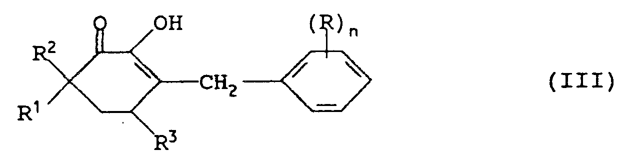 or the general formula 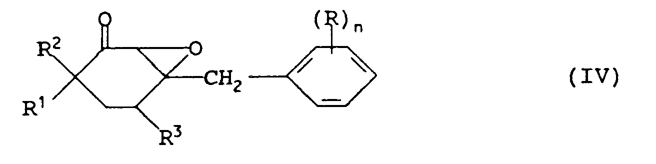 in which n, R, R¹, R² and R³ are as defined in claim 1, with an alkali metal hydroxide in the presence of a polar solvent.

5. A process according to claim 4 in which the polar solvent is a high boiling oligoether or an alcohol.

6. A process or compound according to any preceding claim in which R¹, R² and R³ independently represent a hydrogen atom or a C₁₋₄ alkyl group.

7. A process or compound according to any preceding claim in which R¹, R² and R³ independently represent a hydrogen atom or a methyl group.

8. A process or compound according to any preceding claim in which R represents a halogen atom.

9. A process or compound according to any preceding claim in which n is 1, R represents a chlorine atom, R¹ and R² both represent a hydrogen atom or both represent a methyl group and R³ represents a hydrogen atom or a methyl group.

## Patentansprüche

1. Verfahren zur Herstellung einer Verbindung der allgemeinen Formel 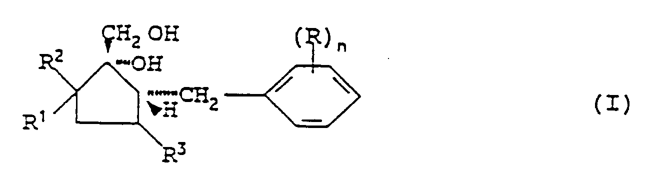 wobei n für eine ganze Zahl von 0 bis 5 steht; jedes R für ein Halogenatom, Nitro-, Cyano-, Hydroxyl-, Alkyl-, Halogenalkyl-, Alkoxy-, Halogenalkoxy-, Amino-, Alkylamino-, Dialkylamino-, Alkoxycarbonyl-, Carboxyl-, Alkanoyl-, Alkylthio-, Alkylsulfinyl-, Alkylsulfonyl-, Carbamoyl-, Alkylamido-, Cycloalkyl- oder Phenylgruppe steht; und R¹, R² und R³ unabhängig ein Wasserstoffatom oder eine Alkylgruppe bedeuten umfassend die Umsetzung einer Verbindung der allgemeinen Formel 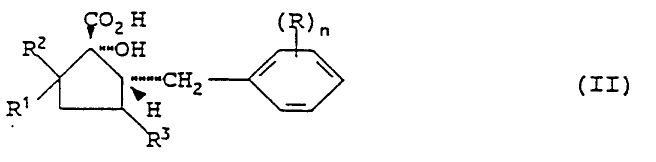 in der n, R R¹, R² und R³ wie oben definiert sind, mit einem Reduktionsmittel.

2. Verfahren gemäß Anspruch 1, wobei das Reduktionsmittel Lithiumaluminiumhydrid oder Natrium-bis(2-methoxyethoxy)aluminiumhydrid ist.

3. Verbindung der allgemeinen Formel II, wie sie in Anspruch 1 definiert ist.

4. Verfahren zur Herstellung einer Verbindung der Formel II, wie sie in Anspruch 1 definiert ist, umfassend das Erhitzen einer Verbindung der allgemeinen Formel 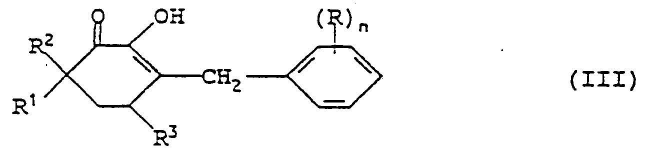 oder der allgemeinen Formel 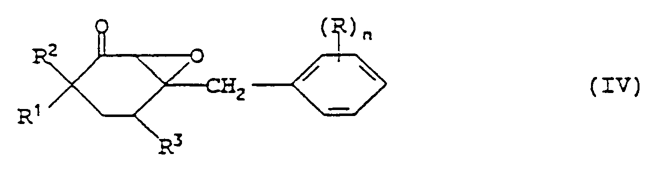 wobei n, R R¹, R² und R³ wie in Anspruch 1 definiert sind, mit einem Alkalimetallhydroxid in Anwesenheit eines polaren Lösungsmittels.

5. Verfahren gemäß Anspruch 4, wobei das polare Lösungsmittel ein hochsiedender Oligoether oder ein Alkohol ist.

6. Verfahren oder Verbindung gemäß einem der vorstehenden Ansprüche, wobei R¹, R² und R³ unabhängig ein Wasserstoffatom oder eine C₁₋₄-Alkylgruppe bedeuten.

7. Verfahren oder Verbindung gemäß einem der vorstehenden Ansprüche, wobei R¹, R² und R³ unabhängig ein Wasserstoffatom oder eine Methylgruppe bedeuten.

8. Verfahren oder Verbindung gemäß einem der vorstehenden Ansprüche, wobei R für ein Halogenatom steht.

9. Verfahren oder Verbindung gemäß einem der vorstehenden Ansprüche, wobei n 1 ist, R für ein Chloratom steht, R¹ und R² beide ein Wasserstoffatom bedeuten oder beide eine Methylgruppe bedeuten und R³ ein Wasserstoff oder eine Methylgruppe bedeutet.

## Revendications

1. Un procédé pour la préparation d'un composé de formule générale 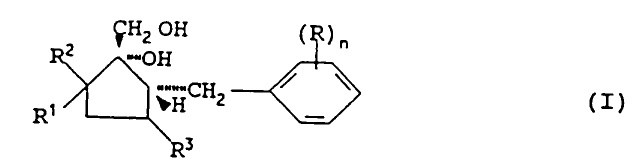 dans laquelle n représente un nombre entier de 0 à 5 ; chaque R représente un atome d'halogène, un groupe nitro, cyano, hydroxyle, alkyle, halogénalkyle, alcoxy, halogénoalcoxy, amino, alkylamino, dialkylamino, alcoxycarbonyle, carboxyle, alcanoyle, alkylthio, alkylsulfinyle, alkylsulfonyle, carbamoyle, alkylamido, cycloalkyle ou phényle ; et R¹, R² et R³ représentent chacun indépendamment un atome d'hydrogène ou un groupe alkyle ; qui consiste à faire réagir un composé de formule générale 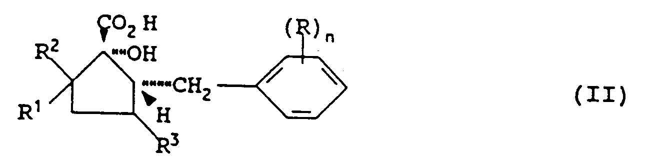 dans laquelle n, R, R¹, R² et R³ sont tels que définis ci-dessus, avec un agent réducteur.

2. Un procédé selon la revendication 1, dans lequel l'agent réducteur est l'hydrure de lithium et d'aluminium ou l'hydrure de sodium et de bis(2-méthoxyéthoxy)aluminium.

3. Un composé de formule générale II tel que défini dans la revendication 1.

4. Un procédé pour la préparation d'un composé de formule II tel que défini dans la revendication 1, qui consiste à chauffer un composé de formule générale 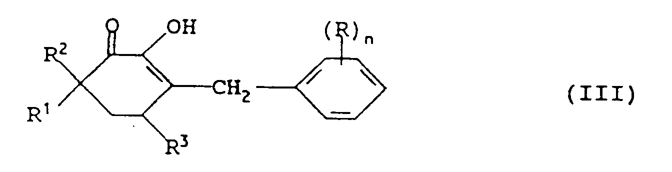 ou de formule générale 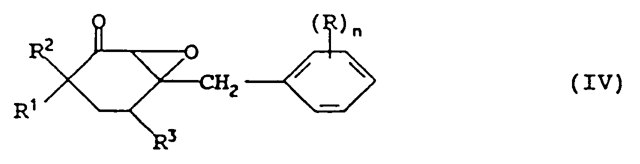 dans laquelle n, R, R¹, R² et R³ sont tels que définis dans la revendication 1, avec un hydroxyde de métal alcalin en présence d'un solvant polaire.

5. Un procédé selon la revendication 4, dans lequel le solvant polaire est un oligoéther à haut point d'ébullition ou un alcool.

6. Un procédé ou un composé selon l'une quelconque des revendications précédentes, dans lequel R¹, R² et R³ représentent chacun indépendamment un atome d'hydrogène ou un groupe alkyle en C₁-C₄.

7. Un procédé ou un composé selon l'une quelconque des revendications précédentes, dans lequel R¹, R² et R³ représentent chacun indépendamment un atome d'hydrogène ou un groupe méthyle.

8. Un procédé ou un composé selon l'une quelconque des revendications précédentes, dans lequel R représente un atome d'halogène.

9. Un procédé ou un composé selon l'une quelconque des revendications précédentes, dans lequel n est 1, R représente un atome de chlore, R¹ et R² représentent chacun un atome d'hydrogène, ou bien représentent chacun un groupe méthyle, et R³ représente un atome d'hydrogène ou un groupe méthyle.
